(19) **Europäisches Patentamt**
**European Patent Office**
**Office européen des brevets**

(11) **EP 3 501 644 A1**

(12) **EUROPEAN PATENT APPLICATION**
published in accordance with Art. 153(4) EPC

(43) Date of publication:
26.06.2019 Bulletin 2019/26

(51) Int Cl.:
*B01J 35/04* (2006.01)  *B01J 32/00* (2006.01)
*B01J 20/32* (2006.01)  *B01J 20/18* (2006.01)
*B01J 20/10* (2006.01)  *B01J 29/06* (2006.01)
*C07C 2/66* (2006.01)  *C07C 7/20* (2006.01)
*B01J 20/08* (2006.01)

(21) Application number: 17841633.5

(22) Date of filing: 03.08.2017

(86) International application number:
PCT/KR2017/008368

(87) International publication number:
WO 2018/034450 (22.02.2018 Gazette 2018/08)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: 16.08.2016  KR 20160103497

(71) Applicant: **Heesung Catalysts Corporation**
**Siheung-si, Gyeonggi-do 15088 (KR)**

(72) Inventors:
• **KIM, Ho-dong**
**Namyangju-si**
**Gyeonggi-do 12085 (KR)**
• **YOO, Young-san**
**Siheung-si**
**Gyeonggi-do 15023 (KR)**
• **HAN, Hyun-sik**
**Seoul 05612 (KR)**

(74) Representative: **Latscha Schöllhorn Partner AG**
**Grellingerstrasse 60**
**4052 Basel (CH)**

(54) **FILTER STRUCTURE AS SOLID CATALYST CARRIER FOR PREPARING ALKYL AROMATIC COMPOUND**

(57) The present invention relates to a support structure of a solid catalyst for preparing a linear alkyl aromatic compound, particularly linear alkylbenzene (LAB), and to a method of preparing an alkyl aromatic compound by alkylating an aromatic compound with an olefin using a solid alkylation catalyst, and of regenerating the deactivated solid alkylation catalyst. The present invention provides an integrated method of preparing an alkyl aromatic compound by alkylating an aromatic compound with an olefin using a filter structure as a solid catalyst carrier for alkylating an aromatic compound with an olefin, and of regenerating the deactivated solid alkylation catalyst, thereby realizing simpler and less expensive processing than conventional processes.

【FIG. 1】

**Description**

**Technical Field**

The present invention relates to a filter structure as a solid catalyst carrier for alkylating an aromatic compound with an olefin, the use thereof, a method of preparing an alkyl aromatic compound using the same, and a method of regenerating a deactivated solid alkylation catalyst.

**Background Art**

The present invention is directed to a support structure of a solid catalyst for preparing a linear alkyl aromatic compound, particularly linear alkylbenzene (LAB). Typically, LAB is prepared by dehydrogenating a linear paraffin to afford a linear olefin, followed by alkylating benzene with the linear olefin in the presence of a homogeneous catalyst such as HF, $AlCl_3$, etc. However, the use of such homogeneous catalysts is increasingly restricted worldwide due to environmental pollution problems, device corrosion, danger upon leakage outside the device, difficulty in separating a product from a catalyst, and the like. Thus, with the goal of replacing the above catalysts, thorough research is ongoing into heterogeneous catalysts, particularly solid-acid catalysts, which are environmentally friendly and non-toxic and have excellent durability and regeneration ability (Hossein F., Comptes Rendus Chimie, 15, 2012, 962). Conventional solid-acid catalysts include clay (Kocal J. A. et al., Appl. Catal. A., 2001, 221: 295), heteropoly acid (C. Hu et al., Appl. Catal. A: Gen. 177, 1999, 237.), zeolite (Cao Y et al., Appl Catal A, 1999, 184: 231), and silica-alumina (US 5,344,997), among which zeolite-related catalysts were commercialized in 1990s by companies such as UOP, Exxon, etc.

Joseph A. Kocal et al. (Appl. Catal., A:Gen., 221 (2001) 295-301) taught that a difference in LAP selectivity depending on the kind of zeolite is caused by acid strength and pore size. U.S. Patent No. 4,395,372 discloses an alkylation process using rare-earth-metal-substituted zeolite X and Y, and U.S. Patent No. 4,876,408 discloses an alkylation process using a catalyst having improved performance through steam treatment of ammonium-substituted zeolite Y.

The prior technique (Korean Patent No. 683509) discloses a method of preparing an alkyl aromatic compound using a solid alkylation catalyst. Here, a solid catalyst is used for the alkylation of an olefin, particularly a monoolefin and an aromatic compound, particularly benzene, but the solid catalyst becomes deactivated over time, and thus there is required means for periodically regenerating the catalyst by removing a gum-type polymer that blocks the reaction sites due to accumulation on the surface of the catalyst during the activation process. The conventional technique proposes a complicated and expensive process in order to introduce the above means. Specifically, during the preparation of LAB, a solid catalyst used for the alkylation of an aromatic compound with an olefin having 6 to 20 carbon atoms is deactivated due to byproducts that are preferentially adsorbed on the catalyst, and these byproducts include $C_{10}$-$C_{20}$ polynuclear hydrocarbons formed during the dehydrogenation of $C_6$-$C_{20}$ linear paraffin, and products having molecular weight larger than desired monoalkyl benzene, for example, dialkyl benzene, trialkyl benzene, and olefin oligomers. Unlike the material that deactivates the catalyst, a catalyst-poisoning material is an aromatic byproduct resulting from the dehydrogenation process, but is known to be easily desorbed from the catalyst when the catalyst is cleaned with an aromatic reactant, particularly benzene (for reference, U.S. Patent No. 5,648,579).

**Disclosure**

**Technical Problem**

In particular, U.S. Patent No. 5,276,231 discloses a process of preparing an alkyl aromatic compound, in which an aromatic byproduct is adsorbed using an adsorbent, and the adsorbent is regenerated through contact with liquid benzene, thereby removing the aromatic byproduct formed during paraffin dehydrogenation.

**Technical Solution**

The present inventors have ascertained that, when using the above regeneration principle and the filter structure specific in the art, means for simplifying the conventional benzene alkylation process may be provided. The present invention addresses a filter structure as a solid catalyst carrier for alkylating an aromatic compound with an olefin. The present invention aims to provide a solid catalyst structure that may be easily and inexpensively mounted in a reactor by implementing the following technical features. The first aspect of the present invention provides a solid catalyst structure for the preparation of an alkyl aromatic compound, particularly a trap-type solid catalyst structure for the preparation of an alkyl aromatic compound, in which pluralities of fluid paths are separated and defined by porous partitions, the inlet side and the outlet side at both ends thereof are sealed in a staggered way with a sealant, the inner surface of each of the partitions communicating with the inlet side is coated with an adsorbent to remove aromatic impurities, and the inner surface of each of the partitions communicating with the outlet side is coated with a solid alkylation catalyst to promote the alkylation of the aromatic compound with an olefin. The second aspect of the present invention provides a solid catalyst structure for the preparation of an alkyl aromatic compound, in which first and second open-type blocks are continuously provided, pluralities of fluid paths of the open-type blocks are separated and defined by porous partitions, the inlet side and the outlet side at both ends thereof are open, the inner surface of each of the partitions of the first open-type block is coated with an adsorbent to remove aromatic impurities, and the inner surface of each of the partitions of the second open-type block is coated with a solid alkylation catalyst to promote the alkylation of the aromatic compound with an olefin.

**Advantageous Effects**

The present invention provides an integrated method of preparing an alkyl aromatic compound by alkylating an aromatic compound with an olefin using a filter structure as a solid catalyst carrier for the alkylation of an aromatic compound with an olefin and of regenerating the deactivated solid alkylation catalyst, thereby realizing simpler and less expensive processing than conventional processes.

**Description of Drawings**

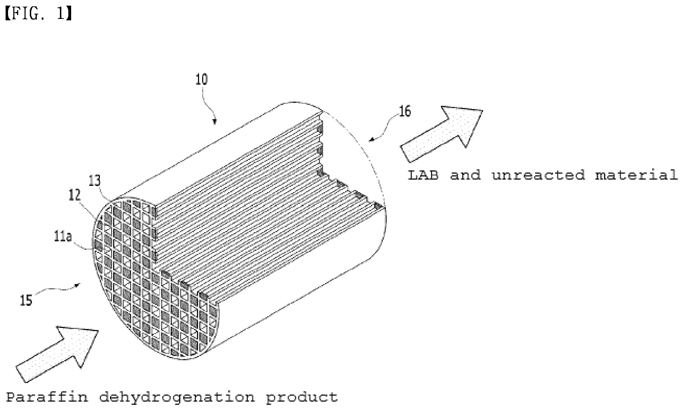

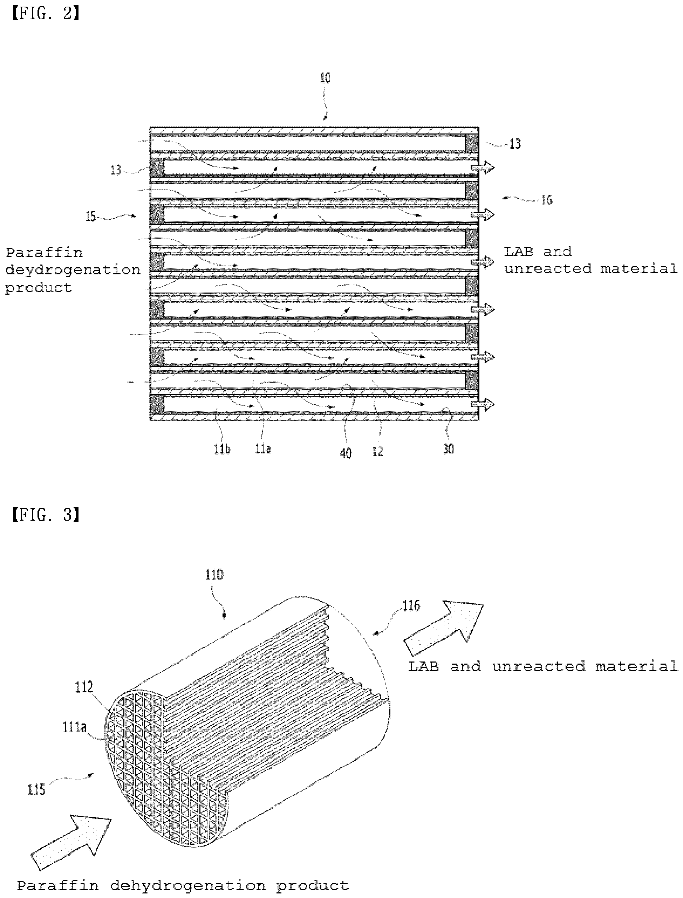

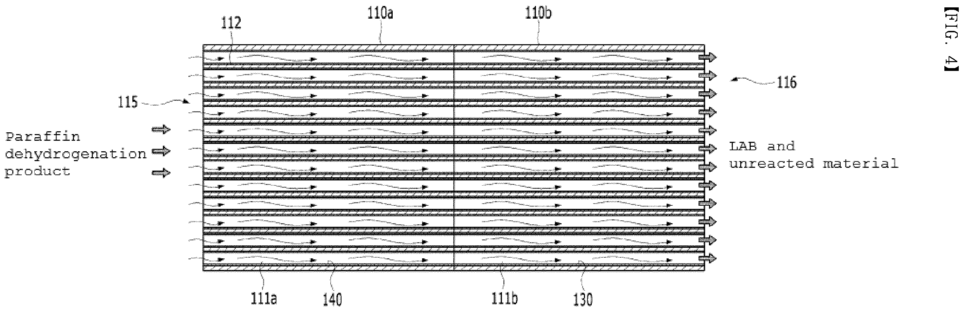

FIG. 1 is a perspective view showing a trap-type catalyst structure according to the present invention; FIG. 2 is a cross-sectional view showing the trap-type catalyst structure according to the present invention; and FIGS. 3 and 4 are a perspective view and a cross-sectional view showing an open-type catalyst structure according to the present invention.

**Best Mode**

The present invention pertains to a support structure of a solid catalyst for the preparation of a linear alkyl aromatic compound, particularly linear alkylbenzene (LAB), and also to a method of preparing an alkyl aromatic compound by alkylating an aromatic compound with an olefin using a solid alkylation catalyst and of regenerating the deactivated solid alkylation catalyst.

The feed supplied to the front end of the catalyst structure according to the present invention is a mixture comprising unreacted paraffin, branched monoolefin, linear monoolefin and impurities, resulting from paraffin dehydrogenation. Such paraffin and monoolefin are typically C6-C22. The monoolefin in the feed reacts with benzene that is additionally supplied, thus producing LAB. However, during the preparation of linear alkylbenzene (LAB), aromatic byproducts or impurities may be typically formed in a dehydrogenation reactor and may act as a material that poisons a benzene alkylation catalyst. When the aromatic byproducts accumulate in an amount of 4 to 10 wt%, the solid alkylation catalyst is rapidly deactivated. In an embodiment of the present invention, aromatic byproducts are removed using the filter structure, and unreacted paraffin, branched monoolefin, and linear monoolefin, from which aromatic byproducts have been removed, are brought into contact with the benzene alkylation catalyst applied on the filter structure so that alkylation progresses, thereby implementing a benzene alkylation process that is simpler and more efficient than conventional processes.

Useful in the present invention, the filter structure has the same configuration as the filter structure of a conventional exhaust gas purification device for diesel engines. Filters are classified into a trap (or wall-flow) type and an open (or straight-flow) type, and both of these structures may be used as a catalyst support in the present invention. As used herein, the terms "catalyst support", "catalyst carrier", and "catalyst support structure" may be interchangeably used, and are to be understood as indicating a structure necessary for maintaining, supporting, applying or coupling a catalyst component. A typical filter structure is formed of a porous silicon carbide sintered body, which is a kind of ceramic sintered body, but as the sintered body other than silicon carbide, a metal material or a sintered body of silicon nitride, SIALON, alumina, cordierite, mullite, etc. may be selected. Regardless of what material is used, a filter catalyst support structure used in the present invention, particularly a trap-type structure described below, is configured such that fine pores are formed in partitions so as to allow target reactants, for example, benzene and monoolefin, to pass through the partitions.

Specifically, a trap-type catalyst support structure 20 is described. A trap-type structure is configured such that pluralities of fluid paths 11a, 11b, the cross-section of which is approximately square-shaped, are separated and defined by thin porous partitions 12, and the inlet side 15 and the outlet side 16 at both ends thereof are sealed in a staggered way with a sealant 13. Thus, the front surface or the rear surface of the trap-type filter structure has a checkerboard pattern. The number of fluid paths is set to about 200 per inch$^2$, and the thickness of the partitions is set to about 0.3 mm. About half of the pluralities of paths are open to the inlet side, and the remaining paths are open to the outlet side.

On the other hand, an open-type catalyst block is a support in which the sealant is not provided at both ends of the trap-type catalyst support structure, and is typically referred to as a straight-flow honeycomb. In the present invention, the open-type catalyst structure 120 is configured such that one block is divided into front and rear sides and subjected to zone coating, but it is to be understood that at least two blocks or supports are provided. Here, the fluid path densities of the two supports may be the same as or different from each other. The structure according to an embodiment of the present invention may be configured such that at least two straight-flow honeycombs are coaxially integrated, or such that a first block and a second block are spaced apart from each other.

With reference to FIGS. 1 and 2, a solid catalyst structure 10 for the preparation of an alkyl aromatic compound according to a first embodiment of the present invention is configured such that pluralities of fluid paths 11a, 11b are separated and defined by porous partitions 12, and the inlet side 15 and the outlet side 16 at both ends thereof are sealed in a staggered way with the sealant 13. Here, the inner surface 40 of each of the partitions communicating with the inlet side is coated with an adsorbent to remove impurities, and the inner surface 30 of each of the partitions communicating with the outlet side is coated with a solid alkylation catalyst to thus promote the alkylation of an aromatic compound with an olefin. With reference to FIG. 3, a solid catalyst structure for the preparation of an alkyl aromatic compound according to a second embodiment of the present invention is configured such that first and second open-type blocks 110a, 110b are continuously provided, pluralities of fluid paths 111a, 111b of the open-type blocks are separated and defined by porous partitions 112, the inlet side 115 and the outlet side 116 at both ends thereof are open, the inner surface 140 of each of the partitions of the first open-type block is coated with an adsorbent to remove impurities, and the inner surface 130 of each of the partitions of the second open-type block is coated with a solid alkylation catalyst to thus promote the alkylation of an aromatic compound with an olefin.

In the first and second embodiments of the present invention, appropriate examples of the adsorbent having selectivity to aromatic byproducts may include a molecular sieve, silica, activated carbon, activated charcoal, activated alumina, silica-alumina, clay, cellulose acetate, synthetic magnesium silicate, porous magnesium silicate and/or porous polystyrene gel. The adsorbent is selected depending on the performance of the adsorbent containing aromatic byproducts, the selectivity of the adsorbent containing aromatic byproducts, which are more harmful to the solid alkylation catalyst described below, etc. The preferred adsorbent is a molecular sieve, and the preferred molecular sieve is 13X zeolite (sodium zeolite X). Also, the solid alkylation catalyst may include a typical solid-acid catalyst, for example, amorphous silica-alumina and a crystalline aluminosilicate material such as zeolite and a molecular sieve. Methods of preparing catalyst components to be applied on the inner surface of the structure and of applying such components may be performed in a manner that is readily understood by those skilled in the art.

Below is a description of a method of preparing an alkyl aromatic compound using the filter structure as the solid catalyst carrier for the preparation of the alkyl aromatic compound and of regenerating the deactivated solid alkylation catalyst, according to the present invention.

Specifically, the feed, comprising unreacted paraffin, branched monoolefin, linear monoolefin and impurities, particularly aromatic impurities, resulting from paraffin dehydrogenation, and benzene are supplied to the inlet side of the solid catalyst structure (trap-type) for the preparation of an alkyl aromatic compound provided in a reactor (not shown). The solid catalyst structure (trap-type) for the preparation of an alkyl aromatic compound is configured such that pluralities of fluid paths 11a, 11b are separated and defined by porous partitions 12, the inlet side 15 and the outlet side 16 at both ends thereof are sealed in a staggered way with a sealant 13, the inner surface of each of the partitions communicating with the inlet side is coated with zeolite 13X, and the inner surface of each of the partitions communicating with the outlet side is coated with a zeolite Y catalyst. Zeolite 13X functions to adsorb aromatic byproducts, which are catalyst-poisoning materials in the feed, and also to allow non-adsorbed pure reactants, such as paraffin, branched monoolefin, and linear monoolefin, to pass through the partitions. The pure reactants passed through the partitions are subjected to alkylation in the presence of the zeolite Y catalyst applied on the opposite side, and a linear alkylbenzene product and an unreacted material are discharged through the outlet side. The linear alkylbenzene product and the unreacted material are separated from each other using downstream columns at the outlet side, and the unreacted material is selectively recirculated to the inlet side of the structure.

The adsorption conditions suitable for the use of zeolite 13X may be selected by those skilled in the art. For example, the adsorption reaction is typically carried out under conditions of a temperature of about 20 to about 300°C, pressure effective for maintaining the stream containing aromatic byproducts in a liquid phase at the selected temperature, and a liquid hourly space velocity of about 1/hr to about 10/hr, and preferably about 1/hr to about 3/hr. Both a liquid-phase process and a vapor-phase process may be used for the adsorptive separation process, but the liquid-phase process may be performed at a low temperature and may exhibit a high adsorption yield of aromatic byproducts resulting therefrom, and is thus preferable. However, the working conditions of the adsorptive separation zone may be optimized by those skilled in the art to operate over a wide range, which is expected to include the conditions in the reaction zone of the present invention and modifications thereof. Meanwhile, the reaction conditions of benzene and linear monoolefin include a temperature ranging from about 80°C to about 160°C. Since the alkylation reaction is carried out through the liquid-phase process, the pressure should be sufficient to maintain the reactants in a liquid phase. The required pressure is inevitably dependent on the feed and the temperature, but is usually set to an absolute pressure of 1480 to 7000 kPa. After a suitable treatment period, the adsorbed aromatic byproducts are removed from the adsorbent and the adsorbent is then regenerated. The adsorbent that is used may be regenerated in a manner of changing the temperature and pressure of the adsorbent or in a manner of removing or desorbing the adsorbed aromatic byproducts through benzene treatment, and preferably, benzene or the unreacted material including the same is allowed to flow backwards from the outlet side to the inlet side, whereby zeolite 13X may be regenerated by desorbing the adsorbed aromatic byproducts therefrom.

**Example 1: Filter-type catalyst structure**

A filter-type catalyst was coated in the following two steps. Specifically, 1,000 g of zeolite 13X (made by Zeolyst), 100 g of boehmite (AlOOH, SASOL), 2,000 g of water, and 35 g of acetic acid (made by Aldrich, 99.9%) were uniformly mixed at room temperature to give a coating solution, which was then uniformly applied on a filter 20 having a cell density of 200 CPSI (cell per square inch) using a spraying process and dried in an oven at 150°C for 12 hr. After drying of the coating solution, thermal treatment was conducted in a firing furnace at 600°C for 6 hr. Thereafter, a solution obtained by uniformly mixing 1,000 g of zeolite Y (made by Albermarle), 100 g of boehmite (AlOOH, SASOL), 2,000 g of water, and 35 g of acetic acid (made by Aldrich, 99.9%) at room temperature was uniformly applied using a spraying process in the opposite direction of the filter, and was then dried in an oven at 150°C for 12 hr. After completion of the drying of the coating solution, thermal treatment was conducted in a firing furnace at 600°C for 6 hr, thereby manufacturing a honeycomb-shaped molded body coated with zeolite 13X and zeolite Y in opposite directions.

**Example 2: Open-type catalyst structure**

Specifically, 1,000 g of zeolite 13X (made by Zeolyst), 100 g of boehmite (AlOOH, SASOL), 2,000 g of water, and 35 g of acetic acid (made by Aldrich, 99.9%) 35 g were uniformly mixed at room temperature to give a coating solution, which was then uniformly applied on the inner surface of a block 121 having a cell density of 200 CPSI (cell per square inch) and dried in an oven at 150°C for 12 hr. After drying of the coating solution, thermal treatment was conducted in a firing furnace at 600°C for 6 hr. Thereafter, a solution obtained by uniformly mixing 1,000 g of zeolite Y (made by Albermarle), 100 g of boehmite (AlOOH, SASOL), 2,000 g of water, and 35 g of acetic acid (made by Aldrich, 99.9%) at room temperature was uniformly applied on the inner surface of a block 122 having a cell density of 200 CPSI (cell per square inch), and then dried in an oven at 150°C for 12 hr. After drying of the coating solution, thermal treatment was conducted in a firing furnace at 600°C for 6 hr. These blocks were brought into contact with each other, thereby manufacturing a catalyst structure.

**Comparative Example 1: Zeolite Y-alone extrudate-type catalyst**

1,600 g of zeolite Y (made by Albermarle) and 400 g of boehmite (AlOOH, SASOL) were mixed, after which 60 g of methylcellulose (made by Aldrich), corresponding to 0.33% of the total weight thereof, was added thereto. Subsequently, a mixed solution comprising 200 g of distilled water and 20 g of nitric acid (made by Aldrich, ~35%) was added to the mixed powder (zeolite Y + boehmite + methylcellulose) and kneaded for 1 hr. After termination of the kneading, an extrudate having a diameter of 1/8 inch was produced using a nozzle-provided single-screw extruder. Thereafter, the extruded catalyst was naturally dried at room temperature for 12 hr, dried at 110°C for 24 hr, and then fired in a firing furnace at 600°C for 4 hr, thus manufacturing a zeolite Y molded body.

**Comparative Example 2: Zeolite 13X extrudate + zeolite Y extrudate**

A zeolite 13x extrudate was manufactured using a typical process, and the zeolite Y molded body of Comparative Example 1 was fed into a reactor. Here, the zeolite 13x extrudate was fed to the front end of the reactor, and the zeolite Y molded body was fed to the rear end of the reactor.

Test Example 1

In order to measure the activity of the catalyst, alkylation was carried out, and a fixed-bed reaction system was used as the reactor. The catalyst prepared in each of Examples and Comparative Examples was loaded in a tubular reactor, and nitrogen gas was allowed to uniformly flow at a rate of 100 cc/min to thus remove air and moisture from the inside of the reactor. Subsequently, the temperature of the reactor was elevated to 130°C, corresponding to the reaction temperature, and maintained, after which the inner pressure of the reactor was uniformly maintained at 10 atm using nitrogen gas by means of a pressure regulator. As the feed used for the reaction, a liquid comprising 1-decene and benzene, mixed at a molar ratio of 1:100, was continuously uniformly supplied to the reactor using an HPLC pump, and the liquid hourly space velocity was uniformly fixed to 1.0 h$^{-1}$. During the reaction for 10 hr, the resulting product was cooled to a temperature of 4°C or less and stored. Thereafter, the product was transferred to a gas chromatographer, followed by quantitative analysis using an FID (flame ionization detector).

The product was calculated for 1-decene conversion, LAB selectivity and LAB yield as follows. The product results using the catalysts are summarized in Table 1 below.

$$\text{Conversion (\%)} = [\text{mol of 1-decene before reaction}$$
$$- \text{mol of 1-decene after reaction}] / [\text{mol of 1-decene before}$$
$$\text{reaction}] \times 100$$

$$\text{LAB Selectivity (\%)} = [\text{mol of LAB of product}] /$$
$$[\text{mol of product}] \times 100$$

$$\text{LAB Yield (\%)} = [\text{conversion} \times \text{LAB selectivity}]/100$$

The product contains all of monoalkylbenzene (LAB), polyalkylbenzene (heavy alkylate), and a light olefin oligomer (light alkylate).

Test Example 2

The reaction was carried out in the same manner as in Test Example 1. In order to evaluate the durability of each catalyst, a continuous reaction was carried out for 100 hr for each catalyst, after which regeneration and then re-reaction were performed. The results are summarized in Table 2 below.

[Table 1]

| No. | Decene Conversion (%) | L't Selectivity (%) | LAB Selectivity (%) | Hvy. Selectivity (%) | LAB Yield (%) |
|---|---|---|---|---|---|
| Ex.1 | 99.9 | 0.04 | 96.7 | 3.26 | 96.6 |
| Ex.2 | 99.9 | 0.06 | 96.3 | 3.64 | 96.2 |
| C.Ex.1 | 99.9 | 0.31 | 96.5 | 3.19 | 96.4 |
| C.Ex.2 | 99.9 | 0.06 | 96.7 | 3.24 | 96.6 |

[Table 2]

| No. | Decene Conversion after 100 hr (%) | Decene Conversion after regeneration (%) | LAB Selectivity after 100 hr (%) | LAB Selectivity after regeneration (%) |
|---|---|---|---|---|
| Ex.1 | 99.2 | 99.9 | 96.8 | 96.8 |
| Ex.2 | 99.1 | 99.9 | 96.5 | 97.1 |
| C.Ex.1 | 92.5 | 99.8 | 96.7 | 96.6 |
| C.Ex.2 | 99.2 | 99.9 | 96.7 | 96.8 |

When the reaction was carried out for 10 hr, each of the catalysts exhibited high decene conversion of 99.9% and LAB selectivity of 96% or more. The main catalyst for the alkylation was zeolite Y, and Examples 1 and 2 and Comparative Example 2 showed high activity by adsorbing and removing the aromatic impurities in the reactants in advance by means of the zeolite 13X layer. Comparative Example 1 showed the same results as those of Examples and Comparative Example 2 despite the fact that the aromatic impurities were not removed because pore clogging due to the byproducts generated during the reaction did not occur for the short reaction time by virtue of the macropores of zeolite Y. After reaction for 100 hr, the catalysts of Examples 1 and 2 and Comparative Example 2 still exhibited the same activity, whereas Comparative Example 1, having no zeolite 13X adsorbent, resulted in a drastic decrease in conversion. The reason why the conversion is lowered in the state in which the selectivity is the same in Comparative Example 1 is that the physical properties of the catalyst are changed. It is considered that the aromatic impurities contained in the reactants have been incorporated into the catalyst, thus blocking the pore openings and reducing the number of zeolite Y surface active sites for a given amount of reactant, resulting in lowered conversion. Commercially, when the conversion is 98% or less and the LAB selectivity is 90% or less, production stops (Korean Patent Application No. 199600006255). Therefore, as in Comparative Example 1, the zeolite Y-alone catalyst is unsatisfactory in terms of reaction durability and the number of regeneration processes is also remarkably increased, thus negating economic benefits. Additionally, when a catalyst that had been used to catalyze a reaction for 100 hr was regenerated and subjected to reaction testing, the initial activity of all four catalysts was restored, from which it can be seen that the aromatic compounds and byproducts incorporated into the catalyst were effectively desorbed.

Based on the above catalyst reaction results, the alkylation reaction according to the present invention can be concluded to be carried out in a single reactor using a single structure, unlike conventional reactions using two existing materials (adsorbent, alkylation catalyst) in respective reactors, and moreover, it is possible to easily remove the adsorbed material from the catalyst even during regeneration, thereby confirming that the catalyst structure is capable of sufficiently catalyzing an alkylation reaction for a long period of time.

**Claims**

1. A solid catalyst structure for preparing an alkyl aromatic compound, wherein a solid catalyst structure (10) for preparing an alkyl aromatic compound is configured such that pluralities of fluid paths (11a, 11b) are separated and defined by porous partitions (12), an inlet side (15) and an outlet side (16) at both ends thereof are sealed in a staggered way with a sealant (13), an inner surface (40) of each of the partitions communicating with the inlet side is coated with an adsorbent to remove impurities, and an inner surface (30) of each of the partitions communicating with the outlet side is coated with a solid alkylation catalyst to catalyze alkylation of an aromatic compound with an olefin.

2. A solid catalyst structure for preparing an alkyl aromatic compound, wherein the solid catalyst structure for preparing an alkyl aromatic compound is configured such that first and second open-type blocks (110a, 110b) are continuously provided, pluralities of fluid paths (111a, 111b) of the open-type blocks are separated and defined by porous partitions (112), an inlet side (115) and an outlet side (116) at both ends thereof are open, an inner surface (140) of each of the partitions of the first open-type block is coated with an adsorbent to remove impurities, and an inner surface (130) of each of the partitions of the second open-type block is coated with a solid alkylation catalyst to catalyze alkylation of an aromatic compound with an olefin.

3. The solid catalyst structure of claim 1 or 2, wherein the adsorbent is a molecular sieve, silica, activated carbon, activated charcoal, activated alumina, silica-alumina, clay, cellulose acetate, synthetic magnesium silicate, porous magnesium silicate or porous polystyrene gel.

4. The solid catalyst structure of claim 1 or 2, wherein the solid alkylation catalyst is a solid-acid catalyst selected from among amorphous silica-alumina and crystalline aluminosilicate.

【FIG. 1】

【FIG. 2】

10

13

13

16

15

Paraffin
deydrogenation
product

LAB and
unreacted material

11b    11a        40    12        30

【FIG. 3】

110

116

112

111a

115

LAB and unreacted material

Paraffin dehydrogenation product

【FIG. 4】

## INTERNATIONAL SEARCH REPORT

| International application No. |
| --- |
| PCT/KR2017/008368 |

| A. | CLASSIFICATION OF SUBJECT MATTER |
| --- | --- |

*B01J 35/04(2006.01)i, B01J 32/00(2006.01)i, B01J 20/32(2006.01)i, B01J 20/18(2006.01)i, B01J 20/10(2006.01)i, B01J 29/06(2006.01)i, C07C 2/66(2006.01)i, C07C 7/20(2006.01)i, B01J 20/08(2006.01)i*

According to International Patent Classification (IPC) or to both national classification and IPC

| B. | FIELDS SEARCHED |
| --- | --- |

Minimum documentation searched (classification system followed by classification symbols)

B01J 35/04; C07C 15/085; B01D 53/86; B01D 53/94; C07C 2/12; F01N 3/021; B01J 37/02; B01J 23/58; B01J 32/00; B01J 20/32; B01J 20/18; B01J 20/10; B01J 29/06; C07C 2/66; C07C 7/20; B01J 20/08

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: alkyl aromatic compound, solid catalyst structure, fluid path, porous partition wall, absorbent, solid catalyst, coating, honeycomb, carrier

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2013-0130608 A (HYUNDAI MOTOR COMPANY et al.) 02 December 2013 See claims 6-8; and figures 5, 6. | 1,3,4 |
| A | | 2 |
| Y | JP 2010-270120 A (STONE & WEBSTER INC.) 02 December 2010 See claims 6-8. | 1-4 |
| Y | KR 10-0667028 B1 (HEESUNG CATALYSTS CORPORATION) 10 January 2007 See claim 1; and figure 3. | 2 |
| A | KR 10-2014-0033469 A (HALDOR TOPSOE A/S) 18 March 2014 See claims 1-12. | 1-4 |
| A | JP 2014-079755 A (DOW GLOBAL TECHNOLOGIES LLC.) 08 May 2014 See claims 1-10. | 1-4 |

☐ Further documents are listed in the continuation of Box C. ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 07 NOVEMBER 2017 (07.11.2017) | **07 NOVEMBER 2017 (07.11.2017)** |

| Name and mailing address of the ISA/KR | Authorized officer |
| --- | --- |
| Korean Intellectual Property Office Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

International application No.

PCT/KR2017/008368

| Patent document cited in search report | Publication date | Patent family member | Publication date |
|---|---|---|---|
| KR 10-2013-0130608 A | 02/12/2013 | CN 103028402 A | 10/04/2013 |
| | | CN 103028402 B | 03/05/2017 |
| | | KR 10-1382686 B1 | 10/04/2014 |
| | | US 2013-0089471 A1 | 11/04/2013 |
| | | US 2013-0089472 A1 | 11/04/2013 |
| | | US 2015-0343381 A1 | 03/12/2015 |
| | | US 2016-0008760 A1 | 14/01/2016 |
| | | US 9108155 B2 | 18/08/2015 |
| | | US 9155999 B2 | 13/10/2015 |
| | | US 9358504 B2 | 07/06/2016 |
| | | US 9527032 B2 | 27/12/2016 |
| JP 2010-270120 A | 02/12/2010 | AU 2003-217760 A1 | 16/09/2003 |
| | | CN 1639089 A | 13/07/2005 |
| | | EP 1485335 A1 | 15/12/2004 |
| | | EP 1485335 B1 | 08/04/2015 |
| | | JP 2005-519103 A | 30/06/2005 |
| | | US 2005-0143612 A1 | 30/06/2005 |
| | | US 2010-0268008 A1 | 21/10/2010 |
| | | US 7777086 B2 | 17/08/2010 |
| | | WO 03-074452 A1 | 12/09/2003 |
| KR 10-0667028 B1 | 10/01/2007 | EP 1931451 A1 | 18/06/2008 |
| | | EP 1931451 B1 | 21/08/2013 |
| | | US 2009-0255237 A1 | 15/10/2009 |
| | | US 7678348 B2 | 16/03/2010 |
| | | WO 2007-040308 A1 | 12/04/2007 |
| KR 10-2014-0033469 A | 18/03/2014 | BR 112014000711 A2 | 14/02/2017 |
| | | CA 2837917 A1 | 17/01/2013 |
| | | CN 103796757 A | 14/05/2014 |
| | | CN 103796757 B | 17/08/2016 |
| | | EP 2731719 A1 | 21/05/2014 |
| | | JP 2014-525825 A | 02/10/2014 |
| | | JP 6130830 B2 | 17/05/2017 |
| | | MX 2014000500 A | 19/02/2014 |
| | | RU 2014104854 A | 20/08/2015 |
| | | RU 2609025 C2 | 30/01/2017 |
| | | US 2014-0140899 A1 | 22/05/2014 |
| | | WO 2013-007467 A1 | 17/01/2013 |
| JP 2014-079755 A | 08/05/2014 | BR PI0412966 A | 26/09/2006 |
| | | CA 2533697 A1 | 10/03/2005 |
| | | CA 2533697 C | 12/02/2013 |
| | | CA 2794531 A1 | 10/03/2005 |
| | | CN 1845780 A | 11/10/2006 |
| | | CN 1845780 B | 16/06/2010 |
| | | EP 1677896 A2 | 12/07/2006 |
| | | EP 1677896 B1 | 17/04/2013 |

Form PCT/ISA/210 (patent family annex) (January 2015)

**EP 3 501 644 A1**

INTERNATIONAL SEARCH REPORT
Information on patent family members

International application No.

**PCT/KR2017/008368**

| Patent document cited in search report | Publication date | Patent family member | Publication date |
|---|---|---|---|
| | | JP 2007-503983 A | 01/03/2007 |
| | | JP 5782085 B2 | 24/09/2015 |
| | | KR 10-1117039 B1 | 15/03/2012 |
| | | KR 10-1141875 B1 | 08/05/2012 |
| | | KR 10-2006-0083450 A | 20/07/2006 |
| | | KR 10-2011-0091818 A | 12/08/2011 |
| | | RU 2006110029 A | 10/08/2006 |
| | | RU 2350379 C2 | 27/03/2009 |
| | | US 2006-0193757 A1 | 31/08/2006 |
| | | US 8661794 B2 | 04/03/2014 |
| | | WO 2005-021138 A2 | 10/03/2005 |
| | | WO 2005-021138 A3 | 19/05/2005 |
| | | ZA 200600757 B | 30/05/2007 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5344997 A
- US 4395372 A
- US 4876408 A
- KR 683509
- US 5648579 A
- US 5276231 A
- KR 199600006255

**Non-patent literature cited in the description**

- **HOSSEIN F.** *Comptes Rendus Chimie,* 2012, vol. 15, 962
- **KOCAL J. A. et al.** *Appl. Catal. A.,* 2001, vol. 221, 295
- **C. HU et al.** *Appl. Catal. A: Gen.,* 1999, vol. 177, 237
- **CAO Y et al.** *Appl Catal A,* 1999, vol. 184, 231
- **JOSEPH A. KOCAL et al.** *Appl. Catal., A:Gen.,* 2001, vol. 221, 295-301